# EUROPEAN PATENT SPECIFICATION

(11) **EP 1 076 509 B1**
(45) Date of publication and mention of the grant of the patent: **22.09.2004**
(21) Application number: 96941550.4
(22) Date of filing: 20.12.1996
(51) Int. Cl.: A61B 5/00, G01N 21/47

(54) **INTEGRATING CAVITY FOR SPECTROSCOPIC MEASUREMENT IN LIGHT SCATTERING SAMPLES**
SPEKTROSKOPISCHE MESSUNGEN AN LICHTSTREUENDEM PRÜFGUT MITTELS INTEGRATIONS -HOHLRAUM
CAVITE INTEGRANTE PERMETTANT D'EFFECTUER DES MESURES DANS DES ECHANTILLONS DISPERSANT LA LUMIERE

(30) Priority: 22.12.1995 GB 9526309
(43) Date of publication of application: 21.02.2001
(73) Proprietor: CME Telemetrix Inc., Waterloo, Ontario N2L 5Z4 (CA)
(72) Inventor: PAWLUCZYK, Romuald, Waterloo, Ontario N2L 5P1 (CA); YANG, Wei, Kitchener, Ontario N2N 3H5 (CA); HOCKLEY, Bernard, S., Georgetown, Ontario L7G 4Z9 (CA)
(74) Representative: Benedum, Ulrich Max, Dr.
(86) International application number: PCT/CA1996/000856
(87) International publication number: WO 1997/023159

(56) References cited:
- WO-A-94/16615
- US-A- 4 768 390
- US-A- 4 867 559
- US-A- 5 372 136

## Description

### BACKGROUND OF INVENTION

### 1. Field of Invention

The field of invention concerns the spectrophotometric determination of the concentration of substances in a light scattering sample. In particular, assessing glucose concentration in tissues of a patient by measuring the near infrared absorption by glucose *in situ* typically using a finger as the tissue sample for analysis.

### 2. Description of Related Art

Measurement of the concentration of components in non-scattering samples and solutions by spectroscopic methods is a well established technique. Spectroscopic measurement usually consists of an optical instrument which generates at least one light beam whose spectral content can be controlled, analyzed and measured in the presence of a sample and can be compared with the spectral content of the same beam in the absence of sample, or other light beam, acting as a reference.

Under optimal conditions, when a detectable optical signal and its spectral content are not affected by any factors other than absorption by the sample which is introduced into a light beam from a light source, spectral variation of the detected optical signal in relation to a reference beam can provide information concerning the chemical composition of the sample. For such determinations it is critical to establish conditions which minimize extraneous factors which affect the signal. This is generally achieved by use of an optical system with a parallel test beam and by using samples in layers of pre-determined thickness. This geometry is intended to assure that the intensity of light detected by a photodetector does not depend on sample position, including small rotations or shift. In use, in circumstances where a distinguishable spectral band is generated by a particular substance's absorption of light, the information can be used to identify that substance and is particularly useful in identifying the substance in samples where the components of the sample, or target, are unknown. Relative variations in absorption spectra may be used to provide information concerning the concentration of the substance.

This technique is based on a number of assumptions. It assumes that the conditions for light propagation remain unchanged, i.e., the light beam does not change geometry. It also assumes that observed losses in light transmitted through the sample are caused only by absorption. In addition, this approach assumes that losses in detected signal depend on the thickness of the absorbing layer and the extinction coefficient (used for mathematical description of absorbance of the analyzed substance) which is proportional to the concentration of the absorber.

These assumptions do not apply in respect of strongly scattering samples or targets. For such targets, two significant factors which affect the light intensity detected by a photodetector are: 1. absorption which affects intensity of light transmitted by the sample, as just discussed, and 2. scattering of light which changes propagation conditions of light. In strongly scattering media, like the majority of biological tissues, even where absorption does not effectively occur, the amount of light which crosses the sample may be very small and its spatial distribution will differ significantly from that of an undisturbed beam. Usually this light is scattered into full solid angle. In such circumstances, the quality and quantity of detected signal greatly depends on the position of the sample in the optical instrument, being sensitive to even minor variations in position. Due to the scattering effect, a portion of the light will leave the sample without penetrating it, while the optical path of light transmitted through the sample may vary across a wide range as a result of numerous changes in direction of particular photons in the sample, all of which leads to considerable difficulty in data interpretation. Depending upon the sample and the conditions for measurement, this may result in a situation where the level of detectable light becomes very low and spectral modulation of intensity is insufficient for recognition of spectral variations of the intensity caused by absorbance by the sample. In circumstances where there is a high concentration of scattering centres (within the sample), back scatter may even prevent light from entering the sample which would result in insufficient interaction of light with the sample and a significant reduction in the amount of light which can reach the photodetector.

Consequently it follows that in the case of targets which strongly scatter light, three central problems to the measurement of characteristic absorption spectra arise:
1. how to cause stronger interaction of a source light with samples to obtain larger spectral intensity modulation;
2. how to collect more light to increase the signal; and
3. how to make the system less sensitive to sample position.

The present invention provides a solution to these problems by application of a light integrating cavity in a novel way. By definition, an integrated cavity is an "emptiness" with non- absorbing strongly scattering walls which scatter light in a uniform fashion. A standard integrating cavity, known as an integrating sphere, has the shape of a sphere with two or three ports depending on the application. One port is used to provide light to the cavity; the second allows light to reach some kind of photodetector (for example, it can be a spectrophotometer); and a third, optional port is provided to secure interaction of light with a target. There can be variations in respect of the cavity including, shape of the cavity, position of the light source inside or outside the cavity, position of the target and the photodetector. This kind of approach is well developed theoretically and has been well described in the literature, for example: "Theory of the Integrating Sphere" by J. A. Jacquez and H. F. Kuppenheim published in JI. Opt. Soc. Amer., 1955, Vol. 45, No. 6, pp. 460-470; or "Incomplete Integrating Sphere" by O. E. Miller and A. J. Stuart published in JI. Opt. Soc. Amer., 1958, Vol. 48, No. 11, pp. 828-831, both of which provide theoretical background to integrating spheres, which to some extent can be expanded to cavities of more general shape. Application of such generalized approaches for spectroscopic measurement is discussed in the paper: "Integrating Cavity Spectroscopy" by P. Elterman published in "Applied Optics", 1970, Vol. 9, No. 9, pp. 2140-2142. A sophisticated variant of the integrating cavity applied to spectroscopic methods is given in a paper "Integrating Cavity Absorption Meter" by E. S. Fry, G. W. Kattawar and R. M. Pope published in Applied Optics, 1992, Vol. 31, No. 12, pp. 2055-2065. The common goal of all these publications is to conduct absolute measurements of absorbance of various targets, light scattering targets in particular. In every case, in order to measure light absorbance, it is believed that the principal property of the cavity is used, i.e., to generate uniform and isotropic irradiance inside the cavity. A theoretical analysis of how a cavity achieves the results it does suggests that such conditions can be achieved only when the surface area of all holes or ports in the cavity wall are small (relative to the size of the cavity) that there is no direct coupling of light from the source to the other holes or ports. Further, if a light absorbing target, or sample, is placed inside a cavity, its volume or extinction coefficient must be small. In other words, absorption of light by a target in a single pass must be "acceptably small". The term "small" as used here means small enough to minimally affect the uniformity of irradiance in the cavity. In addition a sample must be positioned in such a way that light from a source cannot reach it directly, otherwise, nonuniform irradiance of the target is observed, and this is contrary to the requirement of uniform illumination. Consequently, use of an integrating cavity to measure absorption by a light scattering target requires a small target and that it be placed inside a cavity in a way so as to prevent direct coupling of light from a light output hole, or source, and target. In such cases the amount of light which has interacted with the sample reaching a photodetector is very small in comparison with light which has not undergone interaction with the sample. This significantly reduces the dynamic range of the system, hence, reducing the chance of detecting such signal reduction. Dynamic range can be increased by reducing the amount of light which reaches the photodetector without interacting with the sample. Practically this may be achieved by filling the cavity with sample.

In particular, this has been achieved for liquid or gaseous samples by applying a more complex approach to obtain uniform illumination of larger samples, however such samples must be, from all aspects, of a size and shape which is commensurate with the cavity. It is possible to produce such samples in the cases of gases and liquids (which can assume the shape of the container used), and sometimes it is possible to produce solid samples of such shape and size. An ingenious way to use an integrating cavity for absorption measurement of liquids is given in the above-mentioned publication "Integrating Cavity Absorption Meter" by E. S. Fry, G. W. Kattawar and R. M. Pope. In this case, to improve uniformity of the illumination, a cavity with semitransparent diffusely scattering walls was filled with the test liquid and was placed inside another cavity. The walls of the first cavity could be considered as an additional integrating cavity, thus, in totality it could be considered as three cavities, one inside the other. The role of the external cavity is to redistribute available light around the walls of the internal cavity as far as possible. The wall of this cavity, made of strongly scattering material, could be considered as a second cavity whose role is to secure uniform illumination of the second internal cavity which contained the test liquid (it could be gas or a specially prepared solid sample which totally filled the cavity). The irradiance in each of these three cavities was tested by means of fibre optic probes whose tips were inserted into corresponding cavities including that containing the sample, where the tip of the fibre optic sensor was in the sample (test liquid). It was shown that the relative strength of the detected signals depended on the absorption coefficient of the liquid, and, after suitable calibration this information could be used to determine the absorption coefficient of the liquid. The method assumes that irradiance distribution, at least in the internal cavity and in its walls (the second cavity) is isotropic and homogeneous. This assumption is valid only when the absorption coefficient is low. Altogether, this means that the method can only be applied for absorption measurement of weakly absorbing samples, which completely fill the internal cavity, and which will accept the tip of a fibre optic probe. It is obvious that application of this kind of method to solid targets would be very limited and it would be of little use in applications involving living creatures or parts of their anatomy which cannot be formed to the shape of the cavity and, preferably, would not accept insertion of any external probe.

US-A-4 768 390 discloses an apparatus for measuring the photosynthetic activities of a leaf in vivo comprising a spherical chamber for enclosing the leaf, light emitting diode means for illuminating the leaf within the chamber, detector means for detecting modulated fluorescence emission from the leaf. It is emphasised in that document that it is important that the cavity is spherical. There is only one port for transmitting the radiation from the source into the cavity.

US-A-4 867 559 is directed to an apparatus for measuring the fluorescence and absorbance of a liquid sample. That document also shows a spherical integrating cavity having a single radiation input port.

WO-A-9 416 615 shows a system for examination of a volume of biological tissue, the system including a hollow cylinder filled with a medium that surrounds, for example, a finger and prevents escape of introduced photons.

The problem of the present invention is to provide an apparatus and a method including an integrating cavity which also can be used in relation to living tissues, in particular parts of the human body, where neither size or shape can be changed, and which provide a strong measurement signal. The above mentioned problem is solved by an apparatus according to claim 1 as well as a method according to claim 7 of the present invention. Preferred embodiments are disclosed in the dependent claims.

Thus, while use of an integrating cavity or sphere is attractive, there is a need for some other diagnostic technique which can provide more light and will be less sensitive to position of a target and which can be used in relation to living tissues, parts of human body in particular, where neither size or shape can be changed. We have solved these problems by using an integrating cavity in an uncommon way which can be applied not only to objects like fingers but also to other targets of more complex structure.

The problem is solved by an apparatus according to claim 1 and a method according to claims 7 and 13 of the present invention.

### BRIEF SUMMARY OF INVENTION

The present invention advances the spectrophotometric art by providing an improved means of measuring the concentration of a substance in a light scattering sample. The invention incorporates the use of an integrating cavity with multiple input and output ports which provide better illumination for cavities not completely filled with light scattering samples and consequently make a cavity less susceptible to position of the target in the cavity which is capable of recovering the scattered light from a given sample and re-illuminating the sample with that light thereby enhancing the potential for light absorption: This, in turn, provides a stronger signal and wider dynamic range for measurement of light absorption. In contrast with the prior art, surprisingly, we have found that in order to measure samples we do not require uniform small samples located in a position such that irradiation is by reflected radiation. Equally surprising is the fact that the interior surface of the cavity need not be spherical. Further, although homogeneous isotropic illumination is preferred, it is not assumed or required in order to achieve the beneficial results from using an integrating cavity in generating absorption data. Finally, and most surprisingly, measurements of absorption by a highly light scattering sample are best where the cavity volume and shape are approximately equal to the volume and shape of the sample, as opposed to being spherical in shape.

As mentioned before, in the case of strongly scattering objects two problems arise: how to direct light for more efficient interaction with the target and how to improve its collection efficiency. A theoretical analysis of the way integrating cavities work suggests that under conditions which achieve homogeneous irradiance and distribution of light, the probability of direct coupling of light from a light entry hole to a photodetector, omitting the target, is proportional to the ratio of total volume of the cavity to volume of the target. Thus, for large cavities and small targets, the chance that light will leave the cavity without interaction with the target is very large. This chance is additionally increased if the target itself scatters light strongly. In such cases it may be that even if light reaches the target, it is back scattered to the cavity without penetration of the target and, after that, leaves the cavity without further interaction with the target. This further reduces dynamic range of the instrument and in such situations it becomes critical to increase the chance of interaction of light with the target. This is achieved applying an integrating cavity of a volume which is comparable to the volume of the sample. This increases the chance of light interacting with the target even in the case of strongly scattering samples. However, simply making the cavity of comparable volume to the sample causes the distribution of irradiance inside the cavity to become quite sensitive to the size of the target, its shape, its position in the cavity, and its relative position regarding the light input and output ports. This sensitivity is all but eliminated by application of a multiple port configuration where, instead of single large ports for the light source and photodetector, a multiplicity of smaller ports is used for illumination of the target and data collection. The ports can be distributed on the cavity's surface either in random or in some prescribed fashion depending on the cavity and target shapes. In particular, the multiplicity of ports can be localized in such way that they create some characteristic continuous pattern (a ring, for example) on the surface of the cavity. The target can be illuminated either by all entry ports simultaneously or in any spatial or temporal order. The entry ports can be powered either by a single light source, by independent light sources for each port, or by a set of light sources, each of powering a single or any combination of ports. Any method of light division and light delivery systems (including multiple-furcated optical fibres) can be used to redistribute the light from a light source to selected ports. Light from the output ports can also be analyzed as an entity or in any selected spatial and temporal combination of ports and any method to combine light from various ports can be used. The larger the number of ports and the more uniform the distribution of entry and exit ports on the surface of the cavity, the more uniform is the cavity response to irregular targets. This system allows for multiple passes of radiation through the target and is minimally affected by scattered light and provides significant absorption signal. Consequently, the previous problems of minimal signal which are associated with measurement of absorption of strongly scattering samples, or targets, are minimized in the present invention.

In one application of this technology, namely measuring concentrations of glucose in diabetic patients, the integrating cavity overcomes many of the problems outlined above with respect to the measurement of glucose by providing a method and apparatus for measuring target substances in the blood or tissue of a patient in a non-invasive way which is not dependent on position of the tissue being analyzed yet provides a rapid accurate assessment of the concentration of the target compound. Although one type of application is described here, it is to be understood that this technology can be adapted to measure light absorption in any sample and is particularly useful in applications where the sample to be analyzed has a complex shape, and/or scatters light and/or is difficult to maintain in a immovable fixed position.

In a preferred embodiment, the invention employs the use of an integrating cavity for measurement in fingers. Although, measurement in a finger is described here, any tissue or samples would be susceptible for measurement and an integrating cavity or sphere, or other geometric shape can be employed.

In a further preferred embodiment the integrating cavity is of a convenient size which accepts the insertion of a finger into the space of the cavity with minimal space between the wall of the cavity and the exterior surface of the finger. Multiport illumination of a target and multiport detection together with integrating properties of a cavity create a uniform response independent of shape and optical properties of a target, the directional properties of the light source and the photodetector.

In yet a further embodiment the integrating cavity is a tube-shaped container with multiplicity of holes, or ports penetrating the wall of the container to allow entry of a light and its collection or photo detection, this latter aspect, namely photodetection port, is preferably positioned at the bottom of the container, although any number of ports can be used to collect light, and the surface of the cavity employs a highly light reflective coating or layer.

In a further embodiment the ports penetrating the wall of the cavity are distributed in a random fashion.

In yet a further embodiment the ports penetrating the wall of the cavity are distributed in a characteristic continuous ring pattern.

In still a further embodiment the target can be illuminated by all entry ports simultaneously or in any spatial or temporal order, and the collection of light at the ports with a photodetector can be analyzed in any selected spatial and temporal combination.

In further embodiments the photodetector can be any spectrophotometer, spectrograph or spectrum analyzer, or other instrument capable of photo detection.

In a second type of embodiment, the invention employs the use of an integrating cavity for measurement in a stream of liquid. In this embodiment the cavity is shaped to accept a tube through which liquid will pass. The interior walls of the cavity are in contact with the walls of the tubular vessel, although contact is not necessarily required. At least one photodetector is incorporated and it is located at any selected place in the cavity. At least one light input is positioned at another location along the longitudinal dimension of the cavity. Multiple ports of entry can be used for illumination of the cavity interior.

### BRIEF DESCRIPTION OF THE DRAWINGS

**Figure 1** is a schematic representation incorporating an apparatus of the present invention.
**Figure 2** is a side-view of an integrating cavity demonstrating multiple ports from the exterior to the interior of the cavity.
**Figure 3** illustrates a top-view transverse section of the integrating cavity of **Figure 2**.
**Figure 4** is a transverse section of a reconfigurable multilayer integrating cavity.
**Figure 5** is a cross-section of the reconfigurable multilayer integrating cavity of **Figure 4.**
**Figure 6** is a transverse section of an integrating cavity for measurements in a stream of liquid.
**Figure 7** is a cross-section of a reconfigurable integrating cavity with baffle and insert for sample positioning.

### DESCRIPTION OF THE PREFERRED EMBODIMENT(S)

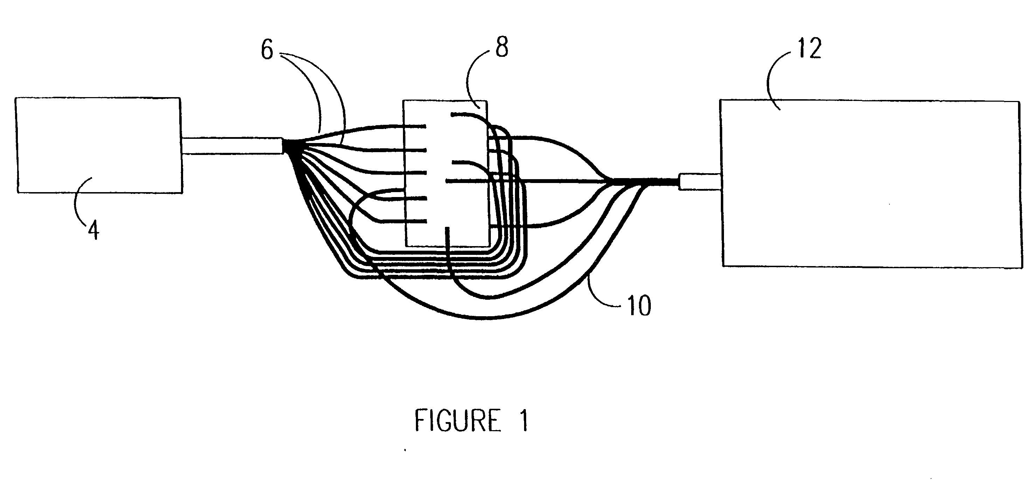

Referring to the figures, **Figure 1**, is a schematic representation of an apparatus **2** in accordance with the present invention. In **Figure 1** there is depicted a radiation emitting source **4**. Radiation from the source is transmitted via fibre-optic bundles **6** to irradiate the interior of an integrating unit **8**. The integrating unit can be an integrating cavity or sphere or similar device capable of receiving illumination and causing a high degree of reflection of the radiation within the interior of the unit. Fibre-optic bundles **10** received radiation from the interior of the integrating unit and transmit it to a detector **12**. The detector can be a spectrograph when radiation source **4** is a broad band light source or, alternatively a photodetector when radiation source **4** is a monochromatic or a tunable laser. The photodetector integrates the optical radiation over a specified time and converts the optical signal to a time multiplexed analog electronic signal called a scan where absorbance is calculated. The electronic signal is amplified by analog electronic amplifiers and converted to a digital signal by an analog-to-digital converter or ADC. The digital information from the converter is interpreted for data analysis by a microprocessor or MP, or other appropriate processor which is in turn connected to a computer. The results of the data analysis can be shown on an output device such as a display and on a printer. A user can control the device to specify a particular interferent to be analyzed and to determine the number and timing of measurements.

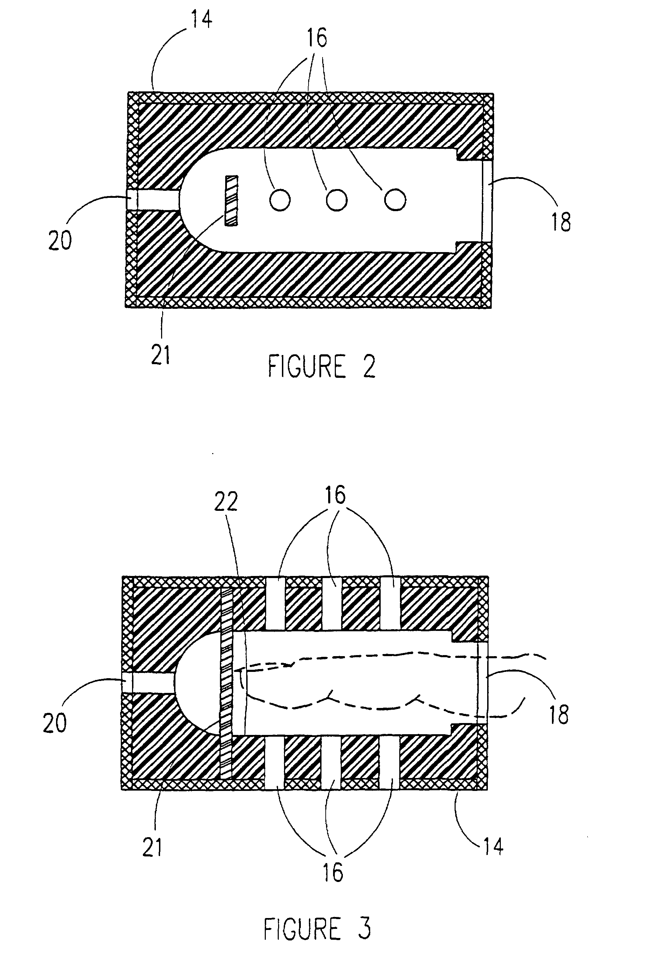

Referring next to **Figure 2,** an example of the integrating unit **2** of **Figure 1** is depicted. Namely, this integrating unit is an integrating cavity **14** with eight (8) (six ports 16, 18, 20) ports that communicate from the exterior to the interior of the cavity. In **Figure 2**, three input ports **16** are illustrated longitudinally linearly located on a first side of cavity **14**. **Figure 3** which provides a different view of cavity **14**, illustrates a further three entry ports **16** located opposite the three entry ports **16** referred to above in respect of **Figure 2**. Ports **16** are multiple input ports which accept fibre-optic bundles from the radiation source and thereby provide illumination to the interior of the cavity. A port **18** at one end of the cavity is of sufficient circumference to allow insertion of a digit or other target while a port **20** positioned opposite entry port **18** is where fibre-optic bundles receive radiation and transport it to a detector **12.** Optionally, the detector can be positioned at the port. Further, while six (6) entry ports and one (1) exit, or detector port are shown, the number of ports can be increased or decreased (to a minimum of one of each) depending upon the circumstances.

The surface of the cavity **22** is made of a material capable of efficient back scattering of illumination such as Spectralon™ or other such material and may be a coating or a layer of such material. The cavity may contain a support **21** for positioning of the tip of a finger within the cavity (there may be more than one and it may appear differently depending on the target). The support is made of, or covered with a material capable of efficient back scattering of illumination such as Spectralon™ or other such material.

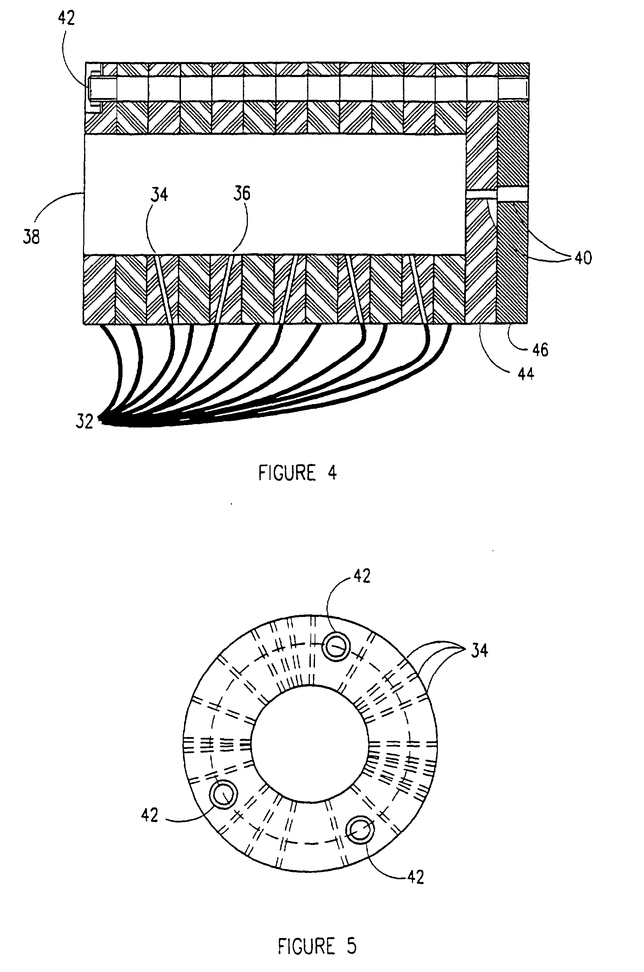

**Figure 4** provides an illustration of another example of integrating unit **8**. This unit is an integrating reconfigurable cavity comprised of a multiple "ring" **32** assembly with variable numbers of input ports **34** that communicate from the exterior to the interior of the cavity for entry of input fibres at various directions. The port for entry of a digit is **38** and the output port is **40**. The embodiment illustrated in **Figure 4** consists of 12 "rings" which are held together by a locking pin **42**, and in this embodiment there are three such pins. Counting from port **34**, the number of input ports, beginning with ring number 3, is 19, followed by a ring with no input ports, which is followed by a ring with 5 input ports followed by a ring with no input ports, which is followed by a ring with 19 input ports, followed by a ring with no input ports, which is followed by a ring with 5 input ports, which is followed by a ring with no input ports which is followed by a ring with 19 input ports, and this is followed by a ring with no input ports, which is followed by a base **44** which contains an exit port **40**. The base is removably fixed to an external base **46** which also contains an exit port **40**, and in which the locking pins are fixed. It is to be understood that the number of rings, the number of entry ports and the number of exit ports as well as their relative positions on the cavity, can all be varied, so long as there is at least one port for the sample to enter, one port for light entry and one port for light to exit.

The rings are made of a material capable of efficient back scattering of illumination such as Spectralon™ or other such material, or the interior of each ring may be covered with a coating or a layer of such material. **Figure 5** provides a cross sectional view and illustrates the three locking pin sites **42,** as well as the exterior to interior input ports **34.** While ring-shaped layers are described here, it is understood that any other shape can be used.

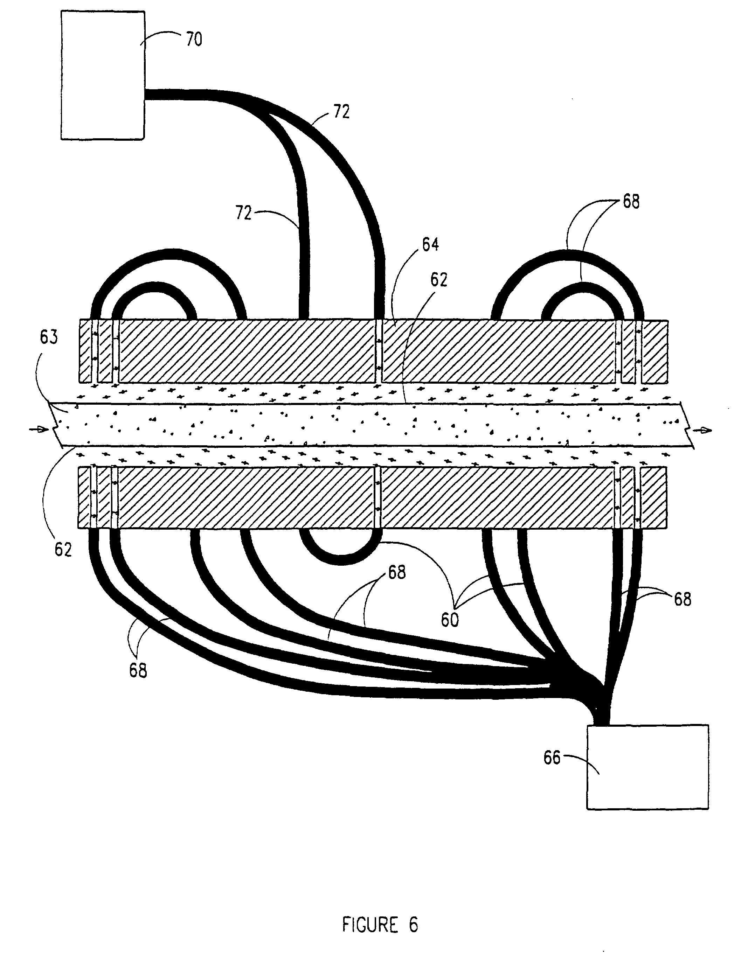

Referring to **Figure 6** a further embodiment of the integrating cavity is shown. This embodiment is intended for use in measuring the concentration of substances in a fluid which is flowing through the interior of the cavity. The integrating cavity **60** optionally contains a tube **62** through which the sample fluid **63** flows. The cavity may be constructed of layers as is the cavity illustrated in **Figure 4**, or it may be, and is preferably of unitary construction as is the cavity illustrated in **Figures 2** and **3**. The walls of the cavity **64** are made of a material capable of efficient back scattering of illumination such as Spectralon™ or other such material, or the interior of the cavity may be covered with a coating or a layer of such material. The radiation source **66** is optically connected to the cavity by fibre optic cables **68** preferably at both ends of the cavity however, input ports can be varied in respect of number of input ports and location along the length of the cavity. A photodetector **70** is optically connected to the cavity by fibre optic cables **72** preferably at the centre of the cavity however, output ports can be varied in respect of number of output ports and location along the length of the cavity. In addition, the input source may be located at the input ports as can the photodetector be located at the output ports.

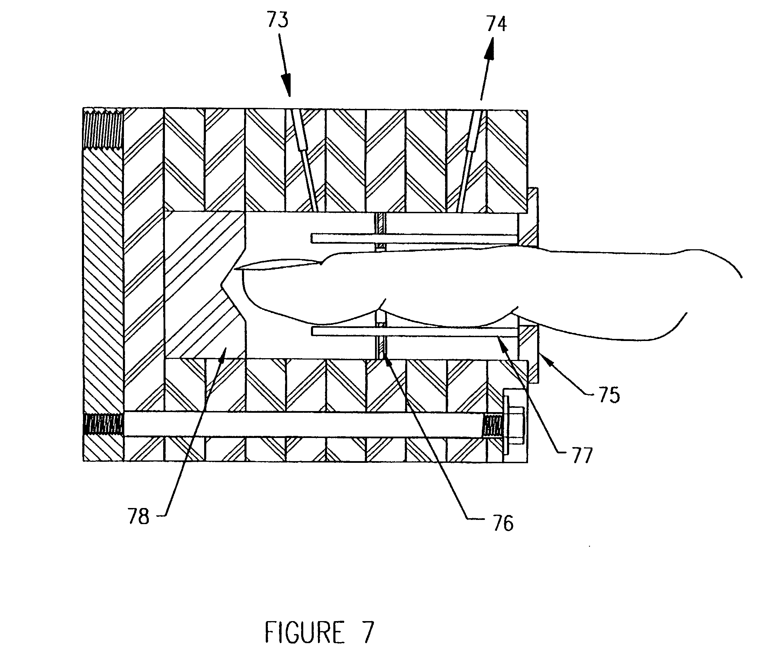

**Figure 7** presents a cross-section of a reconfigurable integrating cavity which has been specifically adapted for a finger sample to be positioned within the cavity. The cavity illustrated has multiple input and output ports respectively (**73** and **74**) and special features to allow immobilization of a finger in position and to reduce light "leak-out" when finger diameter is significantly less than the diameter of the cavity. Collar **75** is made of Spectralon™, or other such material, and assists in preventing light leakage from the cavity. Baffle **76** is also made of Spectralon™ or other such material, and assists in preventing direct light coupling between input and outport ports (**73** and **74** respectively) as well as assisting in holding the finger in position within the cavity. Movement of the finger sample is further prevented by leading rods **77** while the finger tip is positioned in a conical recess at the bottom of the cavity **78**. As is evident and as would be understood by those skilled in the art the sample effect can be achieved with a cavity made from a single piece of light scattering materials or other highly reflecting materials. It should also be understood that any other means which assists in immobilizing the sample for the purposes of measurement are within the scope of the present invention.

While an apparatus which is capable of measuring the concentration of target substances in the blood or tissue of a patient in a non-invasive way which is not dependent on position of the tissue being analyzed yet provides a rapid accurate assessment of the concentration of the target compound is described here, it is to be understood that this technology can be adapted to provide embodiments that measure light absorption in any sample and is particularly useful in applications where the sample to be analyzed has a complex shape, and/or scatters light and/or is difficult to maintain in a immovable fixed position. It may also be adapted to measure the concentration of target substances in a flowing sample of fluid.

It is understood that this disclosure relates to a method of measuring the concentration of target substances in the blood or tissue of a patient in a non-invasive way which is not dependent on position of the tissue being analyzed and providing a rapid accurate assessment of the concentration of the target compound. It is further to be understood that this methodology can be adapted to measure light absorption in any sample and is particularly useful in applications where the sample to be analyzed has a complex shape, and/or scatters light and/or is difficult to maintain in a immovable fixed position. It may also be adapted to measure the concentration of target substances in a flowing sample of fluid.

While the invention has been particularly shown and described with reference to certain embodiments, it will be understood by those skilled in the art that various other changes in form and detail may be made without departing from the scope of the invention as defined in the appended set of claims.

## Claims

1. An apparatus for measurement of a concentration of at least one known substance in a non-uniform sample, said apparatus comprising:
a. a non-spherical integrating cavity (14;64) having an interior and exterior wherein said sample is placed in the interior, said integrating cavity having at least four ports extending from said exterior to said interior;
b. a radiation source (4;66) for emitting a beam of radiation;
c. means (6;68) for transmitting said beam of radiation to the interior of said cavity through at least two of said at least four ports;
d. means (10;72) for receiving radiation from said interior of said integrating cavity through at least two of said at least four ports;
e. a sensor (12;70) optically connected to said interior of said integrating cavity wherein said sensor is responsive to receipt of radiation from said interior of said integrating cavity;
f. means for correlating a sensor response from said sensor to a quantity of at least one known substance in said sample.

2. The apparatus of claim 1, wherein said integrating cavity is cylindrical in shape with an open end and a closed end.

3. The apparatus of claim 2, wherein said integrating cavity (64) comprises a ring structure, the number of rings, entry ports, exit ports as well as their relative positions on the cavity being variable.

4. The apparatus of claim 1, wherein said interior of said integrating cavity is coated with a material capable of efficient back scattering of illumination.

5. The apparatus of claim 1, wherein said interior of said integrating cavity is made of a material capable of efficient back scattering of illumination.

6. The apparatus of claim 3 for the measurement of a concentration of at least one known substance in a human digit, wherein the interior of said integrating cavity consists of a material capable of efficient back scattering of illumination, a collar (75) consisting of a material capable of efficient back scattering of illumination, to partially cover said open end and allowing entry of said digit to the interior of said cavity, a baffle (76) having a perimeter in contact with the interior walls of said cavity and capable of encircling said digit in said cavity and leading rods (77) being provided to help hold said digit in fixed position in said cavity, said at least two ports for the output of a beam of radiation being located above said baffle and said at least two ports for the input of a light beam being located below said baffle, and wherein the apparatus further comprises a spectrophotometer optically coupled to said ports to provide the radiation source and to measure absorbance of radiation by said digit.

7. A method for measurement of concentration of at least one known substance in a non-uniform sample, said method consisting of the following steps:
a. placing said sample into a non-spherical integrating cavity (14;64) having an interior and exterior wherein, said integrating cavity has at least four ports extending from said exterior to said interior;
b. providing a radiation source (4;66) for emitting a beam of radiation;
c. providing means (6;68) for transmitting said beam of radiation to the interior of said cavity through at least two of said at least four ports;
d. providing a means (10;72) for receiving radiation from said interior of said integrating cavity through at least two of said at least four ports;
e. providing a sensor (12;70) optically connected to said interior of said integrating cavity wherein said sensor is responsive to receipt of radiation from said interior of said integrating cavity;
f. correlating a sensor response from said sensor to a quantity of said at least one known substance in said sample.

8. The method of claim 7, wherein said integrating cavity is cylindrical in shape with an open end and a closed end.

9. The method of claim 8, wherein said integrating cavity comprises a ring structure, the number of rings, entry ports, exit ports as well as their relative positions on the cavity being variable.

10. The method of claim 7, wherein said interior of said integrating cavity is coated with a material capable of efficient back scattering of illumination.

11. The method of claim 7, wherein said interior of said integrating cavity is made of a material capable of efficient back scattering of illumination.

12. The method of claim 9 for the measurement of a concentration of at least one known substance in a human digit, wherein the interior of said integrating cavity consists of a material capable of efficient back scattering of illumination, a collar (75) consisting of a material capable of efficient back scattering of illumination being provided which allows entry of said digit to the interior of said cavity, a baffle (76) being provided which has a perimeter in contact with the interior walls of said cavity and is capable of encircling said digit in said cavity, and leading rods (77) being provided to help hold said digit in fixed position in said cavity, the at least two ports for transmitting said beam of radiation to the interior of said cavity being located below said baffle and said at least two ports for outputting said beam of radiation from said interior of said integrating cavity being located above said baffle; and wherein a spectrophotometer optically coupled to said ports provides the radiation source and measures absorbance of radiation by said digit.

13. The use of a spectrophotometric apparatus according to claims 1 to 6 to measure a concentration of at least one known substance in a non-uniform sample.

14. The use of claim 13, wherein said sample is a human digit and said integrating cavity is a cylindrical integrating cavity comprising a ring structure, the number of rings, entry ports, exit ports as well as their relative positions on the cavity being variable, and said cavity has an open end, a closed end, an interior consisting of a material capable of efficient back scattering of illumination, an exterior, a collar consisting of a material capable of efficient back scattering of illumination to partially cover said open end and allowing entry of said digit to the interior of said cavity, a baffle having a perimeter in contact with the interior walls of said cavity and capable of encircling said digit in said cavity, and leading rods to help hold said digit in fixed position in said cavity, and multiple output ports located above said baffle, and multiple input ports located below said baffle.

15. The apparatus of claims 1 and 6, wherein one of said at least one known substance is glucose.

16. The method of claim 7 and 12, wherein one of said at least one known substance is glucose.

17. The use of claims 13 and 14, wherein one of said at least one known substance is glucose.

## Patentansprüche

1. Vorrichtung zur Messung der Konzentration mindestens einer bekannten Substanz in einer nicht-einheitlichen Probe, wobei die Vorrichtung folgendes aufweist:
a. einen nicht-sphärischen Integrationshohlraum (14; 64) mit einem Inneren und einem Äußeren, wobei die Probe im Inneren plaziert wird, wobei der Integrationshohlraum mindestens vier Öffnungen aufweist, die sich vom Äußeren zum Inneren erstrecken;
b. eine Strahlungsquelle (4; 66) zum Emittieren eines Strahlungsbündels;
c. Mittel (6; 68) zum Übertragen des Strahlungsbündels in das Innere des Hohlraumes durch mindestens zwei der mindestens vier Öffnungen;
d. Mittel (10; 72) zum Empfangen von Strahlung aus dem Inneren des Integrationshohlraumes durch mindestens zwei der mindestens vier Öffnungen;
e. einen Sensor (12; 70), der optisch mit dem Inneren des Integrationshohlraumes in Verbindung steht, wobei der Sensor auf den Empfang von Strahlung aus dem Inneren des Integrationshohlraumes anspricht;
f. Mittel zum Korrelieren eines Sensoransprechens durch den Sensor mit einer Menge an mindestens einer bekannten Substanz in der Probe.

2. Vorrichtung nach Anspruch 1, wobei der Integrationshohlraum eine zylindrische Form mit einem offenen Ende und einem geschlossenen Ende aufweist.

3. Vorrichtung nach Anspruch 2, wobei der Integrationshohlraum (64) eine Ringstruktur aufweist, wobei die Anzahl an Ringen, Eintrittsöffnungen, Austrittsöffnungen sowie ihre relativen Positionen auf dem Hohlraum variabel sind.

4. Vorrichtung nach Anspruch 1, wobei das Innere des Integrationshohlraumes mit einem Material beschichtet ist, welches die Bestrahlung effizient zurückstreuen kann.

5. Vorrichtung nach Anspruch 1, wobei das Innere des Integrationshohlraumes aus einem Material hergestellt ist, welches die Bestrahlung effizient zurückstreuen kann.

6. Vorrichtung nach Anspruch 3 für die Messung einer Konzentration mindestens einer bekannten Substanz in einem menschlichen Finger, wobei das Innere des Integrationshohlraumes aus einem Material besteht, welches die Bestrahlung effizient zurückstreuen kann, wobei ein Kragen (75), der aus einem Material besteht, welches die Bestrahlung effizient zurückstreuen kann, um das offene Ende teilweise zu bedecken und das Einführen des Fingers in das Innere des Hohlraums zu ermöglichen, eine Blende (76) mit einem Umfang in Kontakt mit den Innenwänden des Hohlraumes, welche den Finger in dem Hohlraum einschließen kann, und Führungsstäbe (77) vorhanden sind, um beim Halten des Fingers in fixierter Position innerhalb des Hohlraumes zu helfen, wobei die mindestens zwei Öffnungen für den Austritt eines Strahlungsbündels oberhalb der Blende gelegen sind und die mindestens zwei Öffnungen für den Eintritt eines Lichtstrahls unterhalb der Blende gelegen sind, wobei die Vorrichtung weiter ein Spektrophotometer aufweist, das optisch mit den Öffnungen gekoppelt ist, um die Strahlungsquelle bereitzustellen und die Absorption von Strahlung durch den Finger zu messen.

7. Verfahren zum Messen der Konzentration mindestens einer bekannten Substanz in einer nicht-einheitlichen Probe, wobei das Verfahren die folgenden Schritten aufweist:
a. Anordnen der Probe in einem nicht-spärischen Integrationshohlraum (14; 64), welcher ein Inneres und ein Äußeres aufweist, wobei der Integrationshohlraum mindestens vier Öffnungen aufweist, die sich vom Äußeren zum Inneren erstrecken;
b. Bereitstellen einer Strahlungsquelle (4; 66) zum Emittieren eines Strahlungsbündels;
c. Bereitstellen von Mitteln (6; 68) zum Übertragen des Strahlungsbündels in das Innere des Hohlraumes durch mindestens zwei der mindestens vier Öffnungen;
d. Bereitstellen eines Mittels (10; 72) zum Empfangen von Strahlung aus dem Inneren des Integrationshohlraumes durch mindestens zwei der mindestens vier Öffnungen;
e. Bereitstellen eines Sensors (12; 70), der optisch mit dem Inneren des Integrationshohlraumes verbunden ist, wobei der Sensor auf den Empfang von Strahlung aus dem Inneren des Integrationshohlraumes reagiert;
f. Korrelieren eines Sensoransprechens des Sensors auf eine Menge der mindestens einen bekannten Substanz in der Probe.

8. Verfahren nach Anspruch 7, wobei der Integrationshohlraum eine zylindrische Form mit einem offenen Ende und einem geschlossenen Ende aufweist.

9. Verfahren nach Anspruch 8, wobei der Integrationshohlraum eine Ringstruktur aufweist, wobei die Anzahl an Ringen, Eintrittsöffnungen, Austrittsöffnungen sowie ihre relativen Positionen auf dem Hohlraum variabel sind.

10. Verfahren nach Anspruch 7, wobei das Innere um des Integrationshohlraumes mit einem Material beschichtet ist, welches die Bestrahlung effizient zurückstreuen kann.

11. Verfahren nach Anspruch 7, wobei das Innere des Integrationshohlraumes aus einem Material hergestellt ist, welches Bestrahlung effizient zurückstreuen kann.

12. Verfahren nach Anspruch 9 für die Messung einer Konzentration mindestens einer bekannten Substanz in einem menschlichen Finger, wobei das Innere des Integrationshohlraumes aus einem Material besteht, welches Bestrahlung effizient zurückstreuen kann, wobei ein Kragen (75) vorhanden ist, welcher aus einem Material besteht, das Strahlung effizient zurückstreuen kann, und welcher einen Eintritt des Fingers in das Innere des Hohlraums ermöglicht, wobei eine Blende (76) vorhanden ist, welche einen Umfang in Kontakt mit den Innenwänden des Hohlraumes aufweist und den Finger im Hohlraum umschließen kann, und wobei Führungsstäbe (77) vorhanden sind, welche helfen, den Finger im Hohlraum in fixierter Position zu halten, wobei die mindestens zwei Öffnungen zum Übertragen des Strahlungsbündels in das Innere des Hohlraums unterhalb der Blende gelegen sind und die mindestens zwei Öffnungen zum Ausgeben des Strahlungsbündels aus dem Inneren des Integrationshohlraumes oberhalb der der Blende gelegen sind; und wobei ein Spektrophotometer, das optisch mit den Öffnungen gekoppelt ist, die Strahlungsquelle bereitstellt und die Absorption von Strahlung durch den Finger misst.

13. Verwendung einer spektrophotometrischen Vorrichtung nach den Ansprüchen 1 bis 5 zum Messen einer Konzentration mindesteins einer bekannten Substanz in einer nicht-einheitlichen Probe.

14. Verwendung nach Anspruch 13, wobei die Probe ein menschlicher Finger ist und der Integrationshohlraum ein zylindrischer Integrationshohlraum ist, welcher eine Ringstruktur aufweist, wobei die Anzahl an Ringen, Eintrittsöffnungen, Austrittsöffnungen sowie ihre relativen Positionen auf dem Hohlraum variabel sind und wobei der Hohlraum ein offenes Ende, ein geschlossenes Ende, ein Inneres, das aus einem Material besteht, welches Bestrahlung effizient zurückstreuen kann, ein Äußeres, einen Kragen, der aus einem Material besteht, welches Bestrahlung effizient zurückstreuen kann, um das offene Ende teilweise zu bedecken und das Einführen des Fingers in das Innere des Hohlraumes zu ermöglichen, eine Blende mit einem Umfang, der in Kontakt mit den Innenwänden des Hohlraumes steht, die den Finger im Hohlraum umschließen kann, und Führungsstäben zum Unterstützen des Haltens des Fingers in fixierter Position in dem Hohlraum und mehrere Austrittsöffnungen, die sich oberhalb der Blende befinden, und mehrere Eintrittsöffnungen, die sich unterhalb der Blende befinden, aufweist.

15. Vorrichtung nach den Ansprüchen 1 und 6, wobei eine der mindestens einen bekannten Substanz Glucose ist.

16. Verfahren nach Anspruch 7 und 12, wobei eine der mindestens einen bekannten Substanz Glucose ist.

17. Verwendung nach den Ansprüchen 13 und 14, wobei eine der mindestens einen bekannten Substanz Glucose ist.

## Revendications

1. Dispositif de mesure d'une concentration d'au moins une substance connue dans un échantillon non uniforme, ledit dispositif comprenant :
a. une cavité d'intégration non-sphérique (14 ; 64) comprenant un intérieur et un extérieur dans laquelle ledit échantillon est placé à l'intérieur, ladite cavité d'intégration comprenant au moins quatre orifices s'étendant depuis ledit extérieur vers ledit intérieur ;
b. une source de rayonnement (4 ; 66) pour émettre un faisceau de rayonnement ;
c. des moyens (6 ; 68) pour transmettre ledit faisceau de rayonnement vers l'intérieur de ladite cavité à travers au moins deux desdits au moins quatre orifices ;
d. des moyens (10 ; 72) pour recevoir le rayonnement depuis ledit intérieur de ladite cavité d'intégration à travers au moins deux desdits au moins quatre orifices ;
e. un capteur (12 ; 70) raccordé de manière optique audit intérieur de ladite cavité d'intégration dans lequel ledit capteur est réactif à la réception d'un rayonnement provenant dudit intérieur de ladite cavité d'intégration ;
f. des moyens pour corréler une réponse de capteur provenant dudit capteur à une quantité d'au moins une substance connue dans ledit échantillon.

2. Dispositif selon la revendication 1, dans lequel ladite cavité d'intégration est de forme cylindrique avec une extrémité ouverte et une extrémité fermée.

3. Dispositif selon la revendication 2, dans lequel ladite cavité d'intégration (64) comprend une structure en anneau, le nombre d'anneaux, d'orifices d'entrée, d'orifices de sortie ainsi que leurs positions relatives sur la cavité étant variables.

4. Dispositif selon la revendication 1, dans lequel ledit intérieur de ladite cavité d'intégration est recouvert d'un matériau capable d'une rétrodiffusion efficace de l'éclairage.

5. Dispositif selon la revendication 1, dans lequel ledit intérieur de ladite cavité d'intégration est composé d'un matériau capable d'une rétrodiffusion efficace de l'éclairage.

6. Dispositif selon la revendication 3, pour la mesure d'une concentration d'au moins une substance connue dans une empreinte digitale humaine, dans lequel l'intérieur de ladite cavité d'intégration est constitué d'un matériau capable d'une rétrodiffusion efficace de l'éclairage, un collier (75) constitué d'un matériau capable d'une rétrodiffusion efficace de l'éclairage, pour couvrir partiellement ladite extrémité ouverte et permettant l'entrée de ladite empreinte digitale vers l'intérieur de ladite cavité, un écran (76) présentant un périmètre en contact avec les parois intérieures de ladite cavité et capable d'encercler ladite empreinte digitale dans ladite cavité et des tiges de direction (77) étant prévues pour aider à maintenir ladite empreinte digitale dans une position fixe dans ladite cavité, lesdits au moins deux orifices pour la sortie d'un faisceau de rayonnement étant placés au-dessus dudit écran et lesdits au moins deux orifices pour l'entrée d'un faisceau de lumière étant placés au-dessous dudit écran, et dans lequel le dispositif comprend en outre un spectrophotomètre couplé de manière optique auxdits orifices pour fournir la source de rayonnement et pour mesurer l'absorbance de rayonnement de ladite empreinte digitale.

7. Procédé de mesure de concentration d'au moins une substance connue dans un échantillon non-uniforme, ledit procédé comprenant les étapes consistant à :
a. placer ledit échantillon dans une cavité d'intégration non-sphérique (14 ; 64) comprenant un intérieur et un extérieur dans lequel, ladite cavité d'intégration comprend au moins quatre orifices s'étendant depuis ledit extérieur vers ledit intérieur ;
b. fournir une source de rayonnement (4 ; 66) pour émettre un faisceau de rayonnement ;
c. fournir des moyens (6 ; 68) pour transmettre ledit faisceau de rayonnement vers l'intérieur de ladite cavité à travers au moins deux desdits au moins quatre orifices ;
d. fournir des moyens (10 ; 72) pour recevoir le rayonnement depuis ledit intérieur de ladite cavité d'intégration à travers au moins deux desdits au moins quatre orifices ;
e. fournir un capteur (12 ; 70) raccordé de manière optique audit intérieur de ladite cavité d'intégration dans laquelle ledit capteur est réactif à la réception du rayonnement depuis ledit intérieur de ladite cavité d'intégration ;
f. corréler une réponse de capteur provenant dudit capteur à une quantité de ladite au moins une substance connue dans ledit échantillon.

8. Procédé selon la revendication 7, dans lequel ladite cavité d'intégration est de forme cylindrique avec une extrémité ouverte et une extrémité fermée.

9. Procédé selon la revendication 8, dans lequel ladite cavité d'intégration comprend une structure en anneau, le nombre d'anneaux, d'orifices d'entrée, d'orifices de sortie ainsi que leurs positions relatives sur la cavité étant variables.

10. Procédé selon la revendication 7, dans lequel ledit intérieur de ladite cavité d'intégration est recouvert d'un matériau capable d'une rétrodiffusion efficace de l'éclairage.

11. Procédé selon la revendication 7, dans lequel ledit intérieur de ladite cavité d'intégration est composé d'un matériau capable d'une rétrodiffusion efficace de l'éclairage.

12. Procédé selon la revendication 9 pour la mesure d'une concentration d'au moins une substance connue dans une empreinte digitale humaine, dans lequel l'intérieur de ladite cavité d'intégration est constitué d'un matériau capable d'une rétrodiffusion efficace de l'éclairage, un collier (75) constitué d'un matériau capable d'une rétrodiffusion efficace de l'éclairage étant prévu qui permet l'entrée de ladite empreinte digitale vers l'intérieur de ladite cavité, un écran (76) étant fourni qui présente un périmètre en contact avec les parois intérieures de ladite cavité et est capable d'encercler ladite empreinte digitale dans ladite cavité et des tiges de direction (77) étant prévues pour aider à maintenir ladite empreinte digitale dans une position fixe dans ladite cavité, les au moins deux orifices pour transmettre ledit faisceau de rayonnement vers ladite cavité étant placés au-dessous dudit écran et lesdits au moins deux orifices pour émettre ledit faisceau de rayonnement depuis ledit intérieur de ladite cavité d'intégration étant placés au-dessus dudit écran, et dans lequel un spectrophotomètre couplé de manière optique auxdits orifices fournit la source de rayonnement et mesure l'absorbance de rayonnement de ladite empreinte digitale.

13. Utilisation d'un dispositif spectrophotométrique selon les revendications 1 à 6 pour mesurer une concentration d'au moins une substance connue dans un échantillon non-uniforme.

14. Utilisation selon la revendication 13, dans laquelle ledit échantillon est une empreinte digitale humaine et ladite cavité d'intégration est une cavité d'intégration cylindrique comprenant une structure en anneau, le nombre d'anneaux, d'orifices d'entrée, d'orifices de sortie, ainsi que leurs positions relatives sur la cavité étant variables, et ladite cavité comprend une extrémité ouverte, une extrémité fermée, un intérieur constitué d'un matériau capable d'une rétrodiffusion efficace de l'éclairage, un extérieur, un collier constitué d'un matériau capable d'une rétrodiffusion efficace de l'éclairage pour couvrir partiellement ladite extrémité ouverte et permettant l'entrée de ladite empreinte digitale vers l'intérieur de ladite cavité, un écran comprenant un périmètre en contact avec les parois intérieures de ladite cavité et capable d'encercler ladite empreinte digitale dans ladite cavité, et des tiges de direction pour aider à maintenir ladite empreinte digitale dans une position fixe dans ladite cavité, et plusieurs orifices de sortie placés au-dessus dudit écran et plusieurs ports d'entrée placés au-dessous dudit écran.

15. Dispositif selon les revendications 1 à 6, dans lequel une desdites au moins une substance connue est le glucose.

16. Procédé selon les revendications 7 et 12, dans lequel une desdites au moins une substance connue est le glucose.

17. Utilisation selon les revendications 13 et 14, dans laquelle une desdites au moins une substance connue est le glucose.
